# EUROPEAN PATENT SPECIFICATION

(11) **EP 4 364 684 B1**
(45) Date of publication and mention of the grant of the patent: **16.09.2026**
(21) Application number: 22832035.4
(22) Date of filing: 28.06.2022
(51) Int. Cl.: A61B 18/22, A61B 34/20

(54) **MULTI-WAVELENGTH MULTI-CHANNEL LASER SYSTEM FOR THERMAL ABLATION IN NEUROSURGERY**
MEHRKANALIGES LASERSYSTEM MIT MEHREREN WELLENLÄNGEN ZUR THERMISCHEN ABLATION IN DER NEUROCHIRURGIE
SYSTÈME LASER MULTICANAL À LONGUEURS D'ONDE MULTIPLES POUR ABLATION THERMIQUE EN NEUROCHIRURGIE

(30) Priority: 28.06.2021 CN 202110719847
(43) Date of publication of application: 08.05.2024
(73) Proprietor: Hangzhou Genlight Neurotech Co., Ltd., Yuhang District, Hangzhou, Zhejiang 311100 (CN)
(72) Inventor: CAO, Peng, Hangzhou, Zhejiang 311100 (CN); JIN, Huijie, Hangzhou, Zhejiang 311100 (CN); XIA, Liangdao, Hangzhou, Zhejiang 311100 (CN); SHI, Dingsheng, Hangzhou, Zhejiang 311100 (CN)
(74) Representative: Boult Wade Tennant LLP
(86) International application number: PCT/CN2022/101854
(87) International publication number: WO 2023/274221

(56) References cited:
- WO-A1-2021/252122
- CN-A- 102 695 468
- CN-A- 108 836 477
- CN-A- 109 259 858
- CN-A- 113 274 126
- US-A1- 2009 264 730
- US-A1- 2015 087 963
- US-A1- 2015 265 306
- US-A1- 2019 247 120

## Description

### CROSS-REFERENCE TO RELATED APPLICATION

The present disclosure claims priority to Chinese patent application No. 202110719847.5 titled "MULTI-WAVELENGTH LASER SYSTEM FOR THERMAL ABLATION IN NEUROSURGERY" filed on June 28, 2021.

### TECHNICAL FIELD

The present disclosure belongs to the field of laser therapy, and particularly relates to a multi-wavelength laser system for thermal ablation in neurosurgery.

### BACKGROUND OF THE INVENTION

The laser medical technology involves irradiating a laser from a laser device onto a tissue to be treated through a medical optical fiber, and performing lithotripsy or heat coagulation, vaporization or cutting treatment of soft tissues through a thermal effect or shock waves caused by the laser. The laser medical technology is widely applied due to short operation time and relatively small wounds in cell thermal damage, lithotripsy and soft tissue cutting treatment. The MRI-guided laser interstitial thermal therapy (LITT) ("laser thermal therapy" or LITT hereinafter), which has gained attention of medical workers at home and abroad in recent years, makes use of the laser thermal effect principle. This kind of LITT is a percutaneous minimally invasive surgery guided by stereotactic magnetic resonance imaging, in which the laser is transmitted through an optical fiber and acts on a target, so as to selectively ablate diseased tissues. In other words, the optical energy is transmitted to a focal tissue through the optical fiber during the surgery for accurate irradiation and photothermal conversion, so that the focal tissue is thermally coagulated and denatured due to an increased temperature in the focal region, and thereby the treatment purpose is achieved.

Compared with the traditional craniotomy, LITT is considered as a less invasive technique which has shown encouraging results in the treatment of gliomas, brain metastases, radionecrosis and epilepsy, and provides a safer alternative to those patients for whom the lesion cannot be surgically removed, who are not candidates for surgery, or for whom other standard treatment regimens are ineffective. However, this LITT technique has its specific indications, and is only applicable to relatively small and regular lesions for now. In other words, use of this LITT technique is limited by a size and a shape of the lesion to some extent.

Such limitations are caused by the fact that in the existing LITT devices or systems, most laser ablation devices include only a laser device capable of emitting lasers of a single wavelength, and an optical fiber matched with the laser device. In other words, the existing LITT device can only emit lasers of a specific wavelength and have a single light source, and can only perform single-channel ablation. When a focus with an irregular shape is treated, the optical fiber needs to be repeatedly plugged and pulled to achieve multi-track ablation of the whole focus. For example, to ablate a focus with an irregular shape, two ablation tracks are planned in the preoperative planning stage, and when the tissue is ablated with the existing single-wavelength single-channel LITT device, treatment steps including skull perforation, optical fiber puncture, laser ablation and the like need to be performed according to a first ablation track. However, when the ablation is performed along a second ablation track, the operation has to be interrupted to wait for a medical worker to enter the magnetic resonance room or push the patient back to the operating room, to insert an ablation optical fiber into the head of the patient according to the new ablation track, and then a second ablation action is performed. Such actions of interrupting the operation and repeatedly entering the sterile operation environment will undoubtedly increase the operation time and the infection risk of the patient, and should be avoided as much as possible during the surgery. In addition, since the ablation range of the single-wavelength laser is relatively fixed, the ablation of a relatively large focus in a multi-track and multi-ablation mode will inevitably and highly depend on the accuracy of each optical fiber puncture position. Any slight deviation of the optical fiber puncture position is very likely to cause incomplete ablation, and even unnecessary additional remedial puncture and ablation steps. The current single-wavelength single-channel LITT device is characterized by specific requirements in the selection of the focus and a particular approach to surgical planning. In the case of larger foci, this technology typically relies on the precision of optical fiber puncture during the ablation planning process, which is a notable aspect of the LITT technique's operational framework.
D1 (US20150087963A1) discloses an image-guided therapy of a tissue, which can utilize magnetic resonance imaging (MRI) or another medical imaging device to guide an instrument within the tissue. A workstation can actuate movement of the instrument, and can actuate energy emission and/or cooling of the instrument to effect treatment to the tissue.
The instrument can be an MRI compatible laser probe that provides thermal therapy to, e.g., a tissue in a brain of a patient. By tracking a probe position within tissue through feedback, multiple data slices provided around the probe position can be processed to monitor treatment and view thermal data. The operator selects treatment area reference points with the assistance of noise masking.

### SUMMARY OF THE INVENTION

The invention is defined in the appended claims.

### SUMMARY OF THE DISCLOSURE

In some embodiments, the present disclosure is to provide a multi-wavelength laser system for thermal ablation in neurosurgery suitable for a focal tissue, which includes at least two ablation optical fiber catheters, where each optical fiber catheter is connected to a corresponding laser generator, and each laser generator can emit lasers of the same wavelength or different wavelengths. In this manner, the present disclosure allows for use of both a multi-wavelength treatment plan and a high-power single-wavelength single-channel treatment plan for precise conformal ablation on regular or irregular tumors, thereby greatly increasing the application range and use flexibility of LITT.

The technical means adopted by the present disclosure are described below.

In some embodiments, the multi-wavelength laser system of the present disclosure includes: ① A magnetic resonance guidance unit: this unit includes an MRI device and an MRI control center. The MRI control center is configured to perform processing including at least one of: data acquisition, data processing, image reconstruction, image display, or image storage.

② A laser ablation unit: this unit is connected to the magnetic resonance guidance unit and includes a control host. The control host is configured to complete, according to digital image information of a patient, at least one of: profiling, 3D modeling, or generating a surgical plan of the patient. The control host is configured to fuse the digital image information through an MRI temperature imaging technique to generate a real-time temperature image, and display the real-time temperature image on a human-computer interaction module. The laser ablation unit further includes a laser module connected to the control host and provided with N laser devices. N is a positive integer greater than 0. The control host is configured to synchronously or asynchronously regulate laser operation parameters of some or all of the N laser devices according to the surgical plan and the real-time temperature image.

③ An optical fiber catheter unit: this unit is connected to the laser ablation unit and has M optical fiber catheters for ablation. M is a positive integer greater than 0.

In some embodiments, N is greater than or equal to 2.

In some embodiments, M is greater than or equal to 1.

In some embodiments, N is greater than or equal to 2, and when M is equal to N, the optical fiber catheters are connected to the laser devices in one-to-one correspondence to form N ablation channels; and/or the laser module is provided with N laser devices, where N is at least 2; the optical fiber catheter unit is provided with M optical fiber catheters, where M is at least 1; and the N laser devices are connected to each optical fiber catheter, respectively; and/or the laser module is provided with N laser devices, where N is at least 1; the optical fiber catheter unit is provided with M optical fiber catheters, where M is at least 2; and the M optical fiber catheters are connected to each laser device, respectively.

In some embodiments, each laser device is configured to emit lasers of at least one wavelength; and/or the N laser devices all emit lasers of the same wavelength; and/or one part of the N laser devices emit lasers of a first wavelength, and the other part of the N laser devices emits lasers of a second wavelength different from the first wavelength.

In some embodiments, when N is equal to 2, the laser module includes a first laser device and a second laser device; and the control host is configured to synchronously or asynchronously regulate laser operation parameters of the first laser device and the second laser device according to the surgical plan and the real-time temperature image. Meanwhile, the optical fiber catheter unit includes a first optical fiber catheter ("FOFC") and a second optical fiber catheter ("SOFC"); and the first optical fiber catheter is connected to the first laser device, and the second optical fiber catheter is connected to the second laser device.

In some embodiments, at least one of the first optical fiber catheter or the second optical fiber catheter is provided with a temperature measuring optical fiber configured to detect real-time temperatures of the optical fiber catheter unit where the temperature measuring optical fiber is located and a surrounding tissue of the optical fiber catheter unit; the temperature measuring optical fiber is connected to an optical fiber temperature measuring module in the laser ablation unit; and the optical fiber temperature measuring module is connected to a temperature correction module in the control host for temperature correction.

In some embodiments, the temperature correction module is configured to complete processing including at least: taking a real-time temperature value of the tissue acquired by the optical fiber temperature measuring module in real time as a reference temperature measurement value of the magnetic resonance guidance unit, and feeding back a corrected temperature image to the control host. The corrected temperature image is generated by the magnetic resonance guidance unit based on the reference temperature measurement value; and the corrected temperature image fed back to the control host is used to replace the real-time temperature image.

In some embodiments, the control host is configured to perform real-time regulation on the laser module according to the corrected temperature image.

In some embodiments, the first laser device generates lasers of a first wavelength, and the second laser device generates lasers of a second wavelength; the first wavelength and the second wavelength are the same or different; and the laser module has the first laser device that generates the laser of the first wavelength, and the second laser device that generates the laser of the second wavelength.

In some embodiments, the first wavelength is 980nm, and the second wavelength is 1064nm.

In some embodiments, the laser operation parameters include at least one of: a laser output power, laser light emitting time, or a laser light emitting mode.

In some embodiments, the laser module is further configured with a cooling module connected to the control host and the optical fiber catheter unit; and the control host is configured to control operation of the cooling module such that the cooling module cools the optical fiber catheter unit and a surrounding tissue of the optical fiber catheter unit by driving and controlling flow of a cooling medium.

In some embodiments, the optical fiber catheter unit has the first optical fiber catheter and the second optical fiber catheter of the same structure; where the first optical fiber catheter includes an optical fiber, a cooling inner tube and a cooling outer tube.

In some embodiments, the laser ablation unit includes a control host, a human-computer interaction module, a laser module, a cooling module, a power module, an optical fiber temperature measuring module, and an effect evaluation module.

In some embodiments, the power module has at least one UPS device and at least one power distribution control board; where an electric energy input end of the laser ablation unit is connected to a power line; and the power line has one end connected to a commercial power end and the other end connected to the electric energy input end of the UPS device.

In some embodiments, the power distribution control board includes a plurality of connection terminals, a built-in independent switching power supply, and a latching relay; the latching relay is electrically connected to a PCS-1 channel that is configured to be electrically connected to an emergency stop switch and a key switch; and The power distribution control board further includes a PCS-2 channel connected to the cooling module.

In some embodiments, the effect evaluation module is configured to make real-time intraoperative estimates of a tissue ablation condition using an Arrhenius model or a CEM43 model; and
the control host is configured to generate in real time, according to ablation progress fed back by the effect evaluation module, a regulation instruction including at least one of: a laser output power, light emitting time, a light emitting mode, or a coolant flow rate.

In some embodiments, the human-computer interaction module includes a first touch screen, a second touch screen, a third touch screen, an emergency stop switch, a foot pedal controller, a physical button, and an indicator light.

In some embodiments, the first touch screen and the second touch screen are connected to an industrial personal computer of the control host, while the third touch screen is connected to a motherboard of the control host. In addition, the first touch screen includes an MRI real-time temperature monitoring interface and an ablation area evaluation interface; the second touch screen includes a first laser device parameter interface, a second laser device parameter interface, and a cooling module parameter interface; and the third touch screen and the second touch screen display the same or different interface contents.

In some embodiments, the control host is configured to monitor safe operation parameters of the laser module, the optical fiber temperature measuring module and the cooling module in real time; and when an operation parameter exceeds a safe operation threshold value, the control host is configured to control the laser module and/or the cooling module to stop emitting light.

In some embodiments, the multi-wavelength laser system further includes: software, where the software performs functions including at least one of: surgical plan generation, where the surgical plan contains information corresponding to each of the N laser devices, where the information includes at least one of: a planned ablation area or volume, a laser power, light emitting time or a light emitting mode used for achieving a preset ablation result, the number of desired ablation channels, or a cooling flow rate;
real-time control, including under the guidance of the magnetic resonance guidance unit and according to the surgical plan and the corrected temperature image corresponding to each of the N laser devices, regulating operation parameters of each laser device in an operating state and the cooling module in real time, and performing ablation monitoring in real time; or
comparison and analysis, including comparing information in the surgical plan corresponding to each laser device with post-operative information of the laser device, and generating ablation result information according to a comparison result and displaying the ablation result information on the human-computer interaction module; where the compared contents include: a planned ablation area or volume, and an actual post-operative ablation area or volume; and the ablation result information includes at least one of: an ablation area percentage, an ablation volume percentage, an ablation area percentage, or a comparison diagram of before and after ablation.

Compared with the existing art, the present disclosure achieves the following beneficial effects by means of ingenious structural designs:
(1) The present disclosure is suitable for precise conformal ablation on regular or irregular tumors, and not limited by the diameter and shape of focuses, has a higher utilization rate and a wider application range, and enables simultaneous performance of a plurality of ablation operations.
(2) The surgical plan is highly flexible, enabling both a multi-wavelength ablation scheme and a high-power single-wavelength single-channel ablation scheme; and different ablation schemes may be combined into various working modes, thereby achieving random combinations of different laser channels.
(3) The present disclosure can implement simultaneous treatment of a plurality of targets through output aiming at different characteristics of the focus; and the multiple channels may have synchronous or asynchronous working modes. Furthermore, the output wavelength can be selected at will, which may include a single wavelength, or two wavelengths output in an alternative or simultaneous manner.
(4) The unique cooling system can be directly adapted to a normal saline bottle, and a heating device and a backflow detection means are provided to tell whether the cooling system is in normal operation.
(5) The present disclosure includes a multiple control system that can control the laser module and the cooling module by intraoperative software on the second touch screen or a lower computer system on the third touch screen, so that the system is more stable. Meanwhile, a keyboard, a mouse and physical operation buttons are provided so that the risk of a failed touch screen is avoided.
(6) The optical fiber catheter system includes an optical fiber catheter assembly for ablation and an optical fiber catheter fixing assembly, thereby achieving accurate guiding and positioning. The integrated control host system can perform preoperative planning and intraoperative control.
(7) The material property of the temperature measuring optical fiber of the present disclosure meets the requirement of nuclear magnetism compatibility, and the temperature measuring optical fiber can measure temperatures more precisely and stably compared with the existing temperature detector, such as a thermocouple.

### BRIEF DESCRIPTION OF THE DRAWINGS

FIG. 1 is a framework diagram of a multi-wavelength laser system structure according to the present disclosure.
FIG. 2 is a framework diagram of a first example of a multi-wavelength laser system structure according to the present disclosure.
FIG. 3 is a framework diagram of a second example of a multi-wavelength laser system structure according to the present disclosure.
FIG. 4 is a framework diagram of a third example of a multi-wavelength laser system structure according to the present disclosure.
FIG. 5 is a schematic diagram showing connection between a control host and a human-computer interaction module.
FIG. 6 is a schematic structural diagram of a cooling module.
FIG. 7 is a schematic diagram showing connection between a laser module, a cooling module and an optical fiber catheter.
FIG. 8 is a schematic diagram of a multi-wavelength laser system of the present disclosure.
FIG. 9 is a schematic diagram showing power distribution of a power module in a laser ablation unit.
FIGs. 10A to 10H are schematic diagrams showing correspondence between laser modules and optical fiber catheters.
FIGs. 11A to 11C are schematic diagrams of a ring optical fiber, a side-emitting optical fiber and a dispersion optical fiber.

List of reference numerals:
magnetic resonance guidance unit 100
   MRI device 101
   MRI control center 102
laser ablation unit 200
   control host 201
      temperature correction module 2011
      industrial personal computer 2012
      host 2013
   laser module 202
      first laser device 2021
      second laser device 2022
      first cooling module 2023
      second cooling module 2024
   human-computer interaction module 203
      first touch screen 2031
      second touch screen 2032
      third touch screen 2033
      emergency stop switch 2034
      foot pedal controller 2035
      physical button 2036
      indicator light 2037
   power module 204
   key switch 205
   optical fiber temperature measuring module 206
   effect evaluation module 207
optical fiber catheter unit 300
   first optical fiber catheter ("FOFC") 301
   second optical fiber catheter ("SOFC") 302
optical fiber 1
inner tube 2
outer tube 3
coolant inlet 4
coolant outlet 5
backflow cavity 6
fastening assembly 7
temperature measuring optical fiber 8
light emitting part 9
coolant tank 10
   heater 101
   temperature sensor 102
   level sensor 103
peristaltic pump 20
water inlet pipe 30
water return pipe 40
waste tank 50
flow sensor 501.

### DETAIL DESCRIPTION OF THE INVENTION

In order to solve the above-mentioned problems, we have developed a multi-wavelength LITT device or system which can treat multiple regular focal tissues at one time by means of different heat loss and ablation ranges of tissues at different wavelengths. However, due to the limitations of various practical technical conditions, multi-wavelength laser devices are rarely found. There is no high-power multi-wavelength laser treatment instrument available from the manufacturer that can adapt to the requirements of medical applications, especially medical laser applications.

Therefore, it is desirable to provide a new technical solution for the above problems.

The present disclosure provides a multi-wavelength laser system for thermal ablation in neurosurgery, which can enable simultaneous treatment at multiple sites of a focus and adopt different laser parameters for different sites. This multi-wavelength and thermal ablation laser system addresses limitations on the size and shape of the tumor in the existing art, and truly implement precise conformal ablation on any regular or irregular tumor. Meanwhile, the multi-wavelength laser system also provides an optical fiber temperature measuring module, which can monitor a temperature of a tissue in real time, and thereby fill up temperature measurement blanks caused by scanning intervals of the MRI device. The optical fiber temperature measuring module can implement rapid and accurate temperature measurement so that the multi-wavelength laser system can perform accurate temperature measurement and ablation on the tissue even without a cooling module.

The present disclosure will now be described in detail with reference to the drawings and specific embodiments, where the exemplary embodiments and descriptions are merely intended to illustrate the present disclosure, but not to limit the present disclosure. In addition, in order to better explain the present disclosure, the proximal end in the present disclosure refers to an end of the optical fiber light emitting part close to the focal tissue, while an end of the optical fiber light emitting part away from the focal tissue is referred to as a distal end.

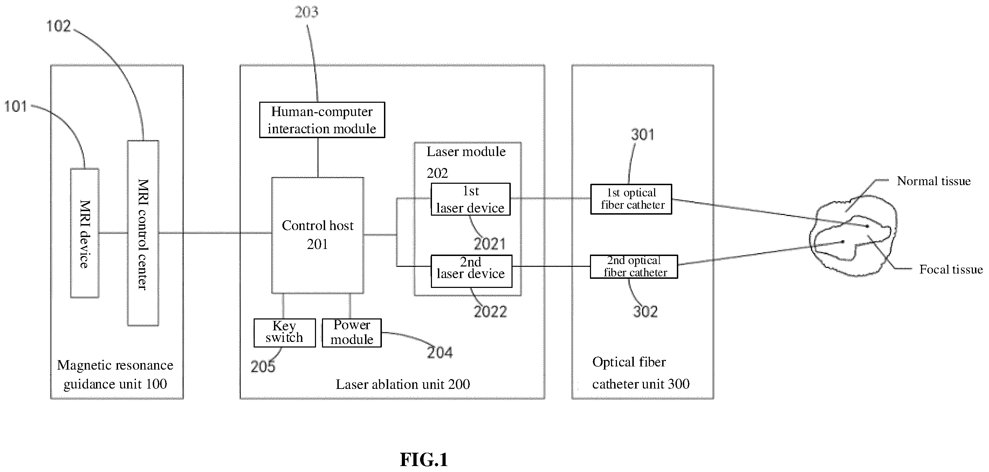

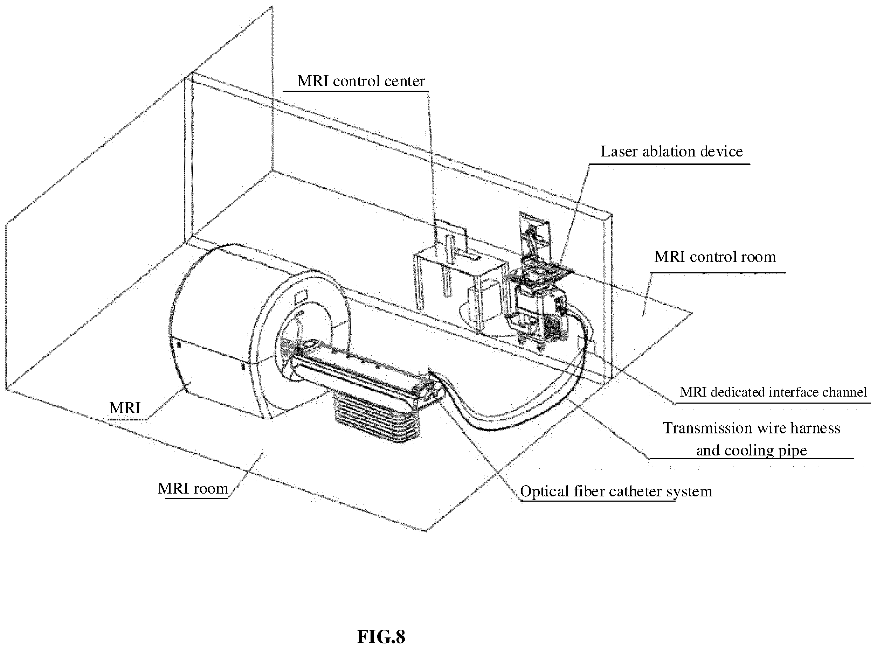

Referring to FIGs. 1 and 8, framework diagrams of a multi-wavelength laser system structure according to the present disclosure are shown. The present disclosure provides a multi-wavelength laser system for thermal ablation in neurosurgery, which mainly includes a magnetic resonance guidance unit 100, a laser ablation unit 200, and an optical fiber catheter unit 300.

[Magnetic resonance guidance unit 100]: the magnetic resonance guidance unit 100 is provided with an MRI device 101 and an MRI control center 102, and the MRI device 101 is connected to the MRI control center 102. The MRI device 101 is configured to monitor medical information of a patient and a tissue of the patient, and transmit the medical information to the MRI control center 102. The MRI control center 102 is configured to perform processing including at least one of: data acquisition, data processing, image reconstruction, image display, or image storage.

[Laser ablation unit 200]: the laser ablation unit 200 is connected to the magnetic resonance guidance unit 100, and includes a control host 201. The control host 201 is configured to complete, according to digital image information of a patient, at least one of: profiling, 3D modeling, or generating a surgical plan of the patient. The control host 201 is configured to fuse the digital image information through an MRI temperature imaging technique to generate a real-time temperature image, and display the real-time temperature image on a human-computer interaction module 203. The digital image information includes, but is not limited to, a CT image, or a magnetic resonance image. The laser ablation unit 200 further includes a laser module 202 connected to the control host 201 and provided with N laser devices. N is a positive integer greater than 0. The control host 201 is configured to synchronously or asynchronously regulate laser operation parameters of the N laser devices according to the surgical plan and the real-time temperature image.

[Optical fiber catheter unit 300]: the optical fiber catheter unit 300 is connected to the laser ablation unit 200 and has M optical fiber catheters for ablation. M is a positive integer greater than 0. The optical fiber catheters are connected to the laser devices in one-to -one correspondence to form M ablation channels.

Further, in some embodiments of the present disclosure, N is greater than or equal to 2.

When N is equal to 2, the laser module 202 includes a first laser device 2021 and a second laser device 2022. The control host 201 is configured to synchronously or asynchronously regulate laser operation parameters of the first laser device 2021 and the second laser device 2022 according to the surgical plan and the real-time temperature image. Meanwhile, the optical fiber catheter unit 300 includes a first optical fiber catheter 301 and a second optical fiber catheter 302. The first optical fiber catheter 301 is connected to the first laser device 2021, and the second optical fiber catheter 302 is connected to the second laser device 2022.

The laser operation parameters include at least one of: a laser output power or light emitting time or light emitting mode, or any combination of the three.

Further, a core point of the laser ablation unit 200 is that the first laser device 2021 generates lasers of a first wavelength, and the second laser device 2022 generates lasers of a second wavelength. The first wavelength and the second wavelength are the same or different.

In some embodiments, the first wavelength is 980nm, and the second wavelength is 1064nm.

In summary, the multi-wavelength laser system has a multi-wavelength laser module capable of synchronously or asynchronously emitting lasers of 980nm and 1064nm, while correspondingly, a first optical fiber catheter and a second optical fiber catheter matched with the laser module are provide to transmit optical energy and ablation. On this basis, the multi-wavelength laser system for thermal ablation in neurosurgery of the present disclosure implements accurate ablation on any tumor by means of the multi-wavelength multi-ablation channel solution, avoids the problem of repeatedly re-plugging the optical fiber for relatively large or irregularly-shaped tumors in the existing art, improves practicability of the device, and provides flexible and varied ablation schemes for medical workers.

Based on the above basic implementation, in some embodiments of the present disclosure, the multi-wavelength laser system for thermal ablation in neurosurgery includes a magnetic resonance guidance unit 100, a laser ablation unit 200, and an optical fiber catheter unit 300. The magnetic resonance guidance unit 100 includes an MRI device 101 and an MRI control center 102. The MRI control center 102 is configured to perform processing including at least one of: data acquisition, data processing, image reconstruction, image display, or image storage.

The laser ablation unit 200 is connected to the magnetic resonance guidance unit 100 and includes a control host 201. The control host 201 is configured to complete, according to digital image information of a patient, at least one of: profiling, 3D modeling, or generating a surgical plan of the patient. The control host 201 is configured to fuse the digital image information through an MRI temperature imaging technique to generate a real-time temperature image, and display the real-time temperature image on a human-computer interaction module 203. The laser ablation unit 200 further includes a laser module 202 connected to the control host 201 and provided with N laser devices. The control host 201 is configured to synchronously or asynchronously regulate laser operation parameters of some or all of the N laser devices according to the surgical plan and the real-time temperature image.

The optical fiber catheter unit 300 is connected to the laser ablation unit 200 and has M optical fiber catheters for ablation. Further, N is greater than or equal to 2, and/or M is greater than or equal to 1.

In order to achieve the purpose of ablating a relatively large focal tissue at one time or completing multi-point ablation in a focal region at one time, an ablation method is further provided for illustration. The flexible selectivity of various ablation channels in the number of the latter generated ablation channels is achieved based on a correspondence connection relationship between the laser module 202 and the optical fiber catheter unit 300, including but not limited to a case where the laser module 202 and the optical fiber catheter unit 300 are connected in one-to -one correspondence, that is, a one-to -one connection form; or a case where a plurality of laser modules 202 are connected to one optical fiber catheter unit 300, that is, a more-to -one connection form; or a case where one laser module 202 is connected to a plurality of optical fiber catheter units 300, that is, a one-to -many connection form; or a case where a plurality of laser modules 202 are connected to a plurality of optical fiber catheter units 300, that is, a many-to -many connection form. Apparently, various flexible variations based on the above correspondence connection relationship may also be included, or variations of the correspondence relationship based on the number of laser devices in the laser module 202 and the number of optical fiber catheters in the optical fiber catheter unit 300 may be included, which are also within the protection scope of the present disclosure. Only some specific ones of the numerous correspondence connection relationships are described below.

### I. A first form of the correspondence connection relationship.

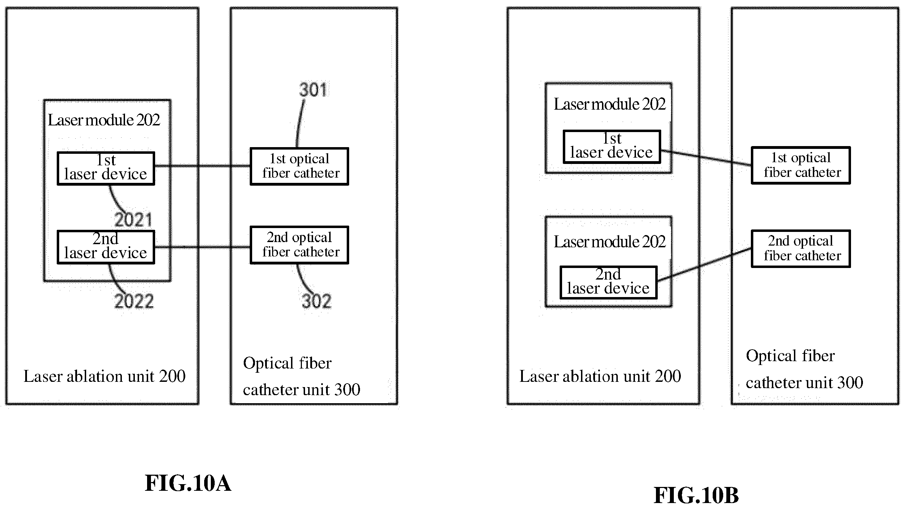

Referring to FIG. 10A, the laser module 202 is provided with N laser devices, and the optical fiber catheter unit 300 has M optical fiber catheters for ablation. Both N and M are positive integers greater than 0. In some embodiments, N is greater than or equal to 2, and M is greater than or equal to 2. Further, when M is equal to N, the optical fiber catheters are connected to the respective laser devices in one-to -one correspondence to form N (or M) ablation channels.

Another variation is shown in FIG. 10B. As shown in FIG. 10B, the laser ablation unit 200 is provided with at least two laser modules 202, and each laser module 202 is provided with at least one laser device. The optical fiber catheter unit 300 is provided with optical fiber catheters of at least the same number as the laser devices. In other words, the optical fiber catheter unit 300 is provided with at least two optical fiber catheters. Referring again to FIG. 10B, a first laser device in a first laser module 202 is connected to the first optical fiber catheter; and a second laser device in a second laser module 202 is connected to the second optical fiber catheter.

### II. A second form of the correspondence connection relationship.

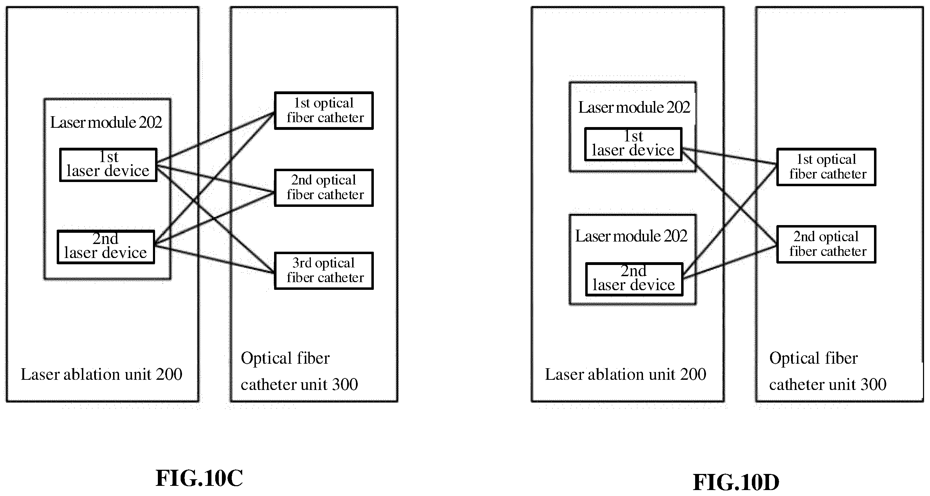

Referring to FIG. 10C, the laser module 202 is provided with N laser devices, where N is at least 2; the optical fiber catheter unit 300 is provided with M optical fiber catheters, where M is at least 1; and the N laser devices are connected to each optical fiber catheter, respectively. Further, as shown in FIG. 10C, N is equal to 2 (including a first laser device and a second laser device), and M is equal to 3 (including a first optical fiber catheter, a second optical fiber catheter, and a third optical fiber catheter). In other words, the first laser device is connected to the first optical fiber catheter, the second optical fiber catheter and the third optical fiber catheter simultaneously or non-simultaneously; and the second laser device is connected to the first optical fiber catheter, the second optical fiber catheter and the third optical fiber catheter simultaneously or non-simultaneously.

Another variation is shown in FIG. 10D: the laser ablation unit 200 is provided with at least two laser modules 202, and each laser module 202 is provided with at least one laser device. The optical fiber catheter unit 300 is provided with optical fiber catheters of at least the same number as, or of a different number from, the laser devices. FIG. 10D shows a scheme in which the optical fiber catheter unit 300 is provided with at least two optical fiber catheters. Therefore, the correspondence connection relationship in this case is that a first laser device in a first laser module 202 is connected to both the first optical fiber catheter and the second optical fiber catheter; and a second laser device in a second laser module 202 is connected to both the first optical fiber catheter and the second optical fiber catheter.

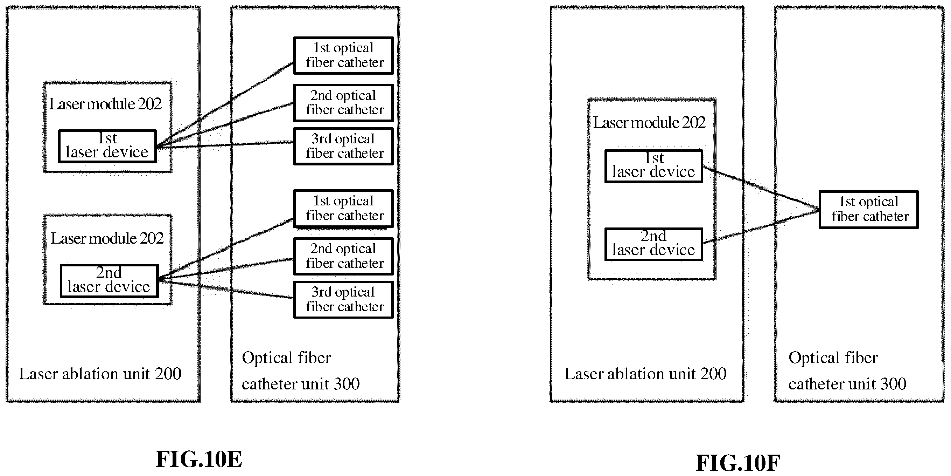

Furthermore, the correspondence connection relationship may be the connection mode shown in FIG. 10E, in which the optical fiber catheter unit 300 is provided with two groups of optical fiber catheters, each including a first optical fiber catheter, a second optical fiber catheter, and a third optical fiber catheter. In other words, the first laser device in the first laser module 202 is connected to the first optical fiber catheter, the second optical fiber catheter and the third optical fiber catheter in a first group of optical fiber catheters simultaneously or non-simultaneously; and the second laser device in the second laser module 202is connected to the first optical fiber catheter, the second optical fiber catheter and the third optical fiber catheter in a second group of optical fiber catheters simultaneously or non-simultaneously.

### III. A third form of the correspondence connection relationship.

Referring to FIG. 10F, the laser module 202 is provided with N laser devices, where N is at least 1; the optical fiber catheter unit 300 is provided with M optical fiber catheters, where M is at least 2; and the M optical fiber catheters are connected to each laser device, respectively. With continued reference to FIG. 10F, a first laser device is connected to a first optical fiber catheter, and a second laser device is connected to the first optical fiber catheter.

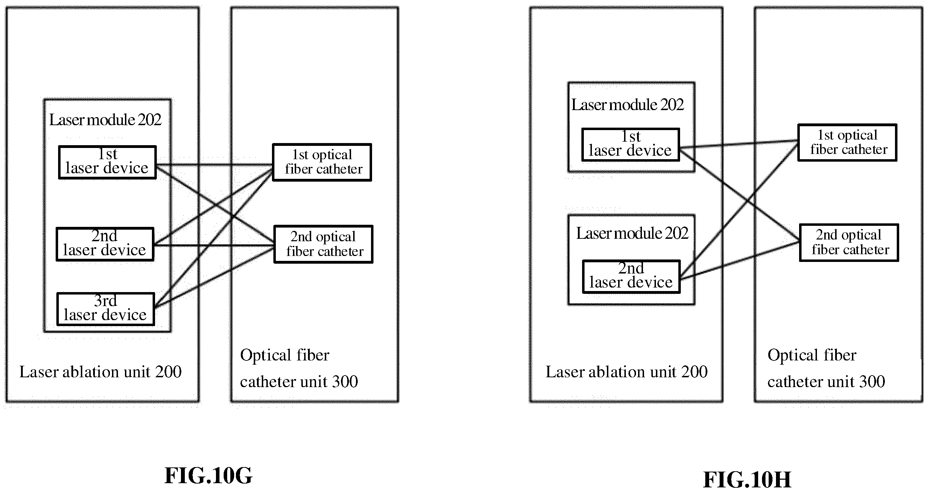

Another form is as shown in FIG. 10G, in which the laser module is provided with a first laser device, a second laser device, and a third laser device, while the optical fiber catheter unit 300 is provided with a first optical fiber catheter and a second optical fiber catheter. The correspondence relationship between the laser module and the optical fiber catheter unit is that: the first laser device is connected to the first optical fiber catheter and the second optical fiber catheter simultaneously or non-simultaneously; the second laser device is connected to the first optical fiber catheter and the second optical fiber catheter simultaneously or non-simultaneously; and the third laser device is connected to the first optical fiber catheter and the second optical fiber catheter simultaneously or non-simultaneously.

Another connection form is shown in FIG. 10H, in which: the laser ablation unit 200 is provided with at least two laser modules 202, and each laser module 202 is provided with at least one laser device. The optical fiber catheter unit 300 is provided with optical fiber catheters of at least the same number as, or of a different number from, the laser devices. In the scheme shown in FIG. 10H, the optical fiber catheter unit 300 is provided with at least a first optical fiber catheter and a second optical fiber catheter. Therefore, the correspondence connection relationship in this case is that a first laser device in a first laser module 202 is connected to both the first optical fiber catheter and the second optical fiber catheter; and a second laser device in a second laser module 202 is connected to both the first optical fiber catheter and the second optical fiber catheter.

On the basis of the above embodiments, the surgical plan of the present disclosure contains information corresponding to each of the N laser devices, where the information includes at least one of: a planned ablation area and/or volume, a laser power, laser light emitting time or a light emitting mode used for achieving a preset ablation result, the number of desired ablation channels (i.e., a selected connection mode between the laser module and the optical fiber catheter), a coolant flow rate, or insertion path planning for an optical fiber catheter. Compared with the existing art, this solution has more flexible selectivity and a wider application range.

It is further preferred that each laser device emits lasers of at least one wavelength. In other words, a laser device may be configured to emit lasers of one wavelength, or emit lasers of two or more wavelengths in the same range or different ranges.

In some embodiments, N laser devices are provided, and each laser device may be configured to emit lasers of only one wavelength; and/or one part of the N laser devices may be each set to emit lasers of only a first wavelength, while the other part of the N laser devices may be each set to emit lasers of a second wavelength different from the first wavelength.

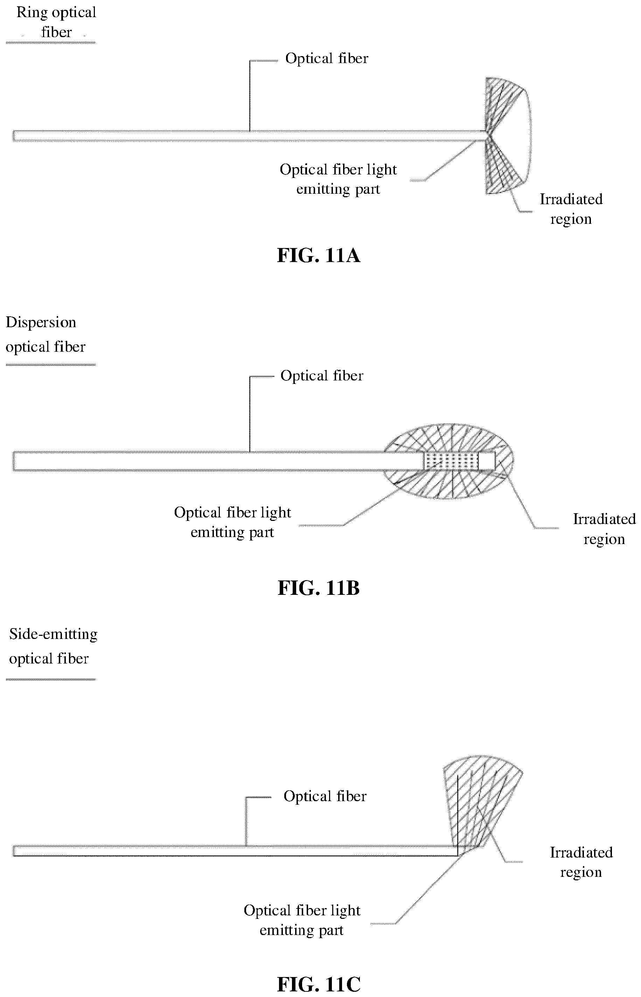

In some embodiments, as shown in FIGs. 11A to 11C, the optical fiber in the optical fiber catheter may include one of a ring optical fiber, a dispersion optical fiber or a side-emitting optical fiber. The ring optical fiber is a laser transmission optical fiber with a light emitting mode of emitting along the whole circumference in the radial direction at a front end of the optical fiber. The dispersion optical fiber is a laser transmission optical fiber with a light emitting mode of emitting along the whole circumference in both radial and axial directions over a predefined length at a front end of the optical fiber. The side-emitting optical fiber is a laser transmission optical fiber with a light emitting mode of emitting from one side in the radial direction at a front end of the optical fiber. A proximal end of the optical fiber (i.e., the end close to the focal tissue) may be further configured as one of a beveled end, a semicircular end, a spherical end, a conical end, or a chisel end.

Apparently, based on the design idea of the present disclosure, various forms of embodiments can be obtained by further optimization, which will be further explained below with reference to the accompanying drawings.

First embodiment: an optimization scheme for accurate temperature measurement design.

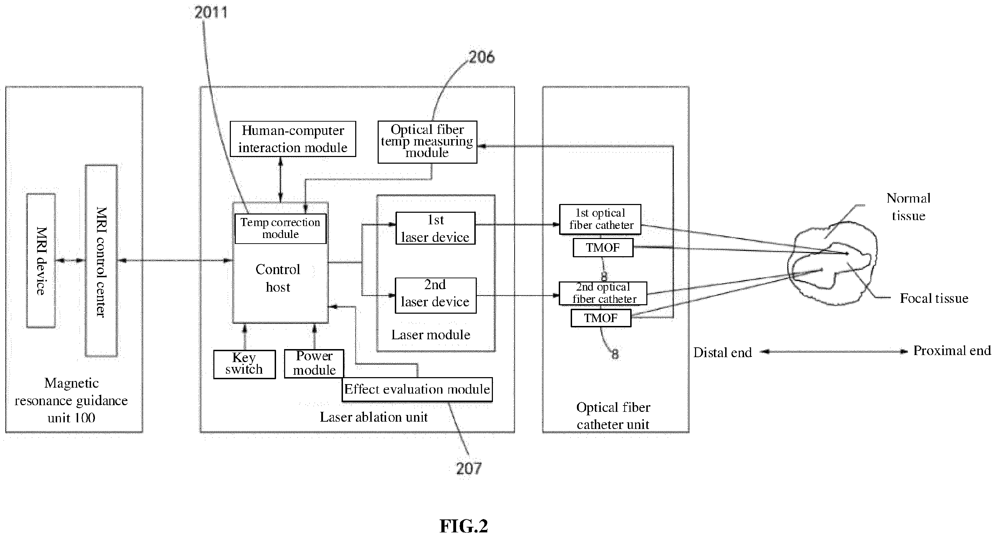

Referring to FIG. 2, on the basis of the multi-wavelength laser system described above, the first embodiment is improved in that the multi-wavelength laser system has both the temperature measurement function of the magnetic resonance guidance unit and the function of detecting a tissue temperature in real time, thereby achieving dual-precise temperature measurement. Specifically, since the first optical fiber catheter 301 and the second optical fiber catheter 302 have the same structure, the first optical fiber catheter 301 is taken as an example here for illustration: the first optical fiber catheter 301 is provided with a temperature measuring optical fiber ("TMOF") 8, which is connected to the first optical fiber catheter 301 by a connection means including, but not limited to, adhesion, embedding arrangement or the like. In some embodiments, a temperature detecting head of the temperature measuring optical fiber 8 is close to a light emitting part 9 of the first optical fiber catheter 301, and a plurality of temperature detecting heads may be arranged along a length direction of the first optical fiber catheter 301.

The temperature measuring optical fiber 8 is configured to detect and acquire real-time temperatures of the first optical fiber catheter 301 where the temperature measuring optical fiber 8 is located and a surrounding tissue of the first optical fiber catheter 301, and transmit data information of the real-time temperatures to the optical fiber temperature measuring module 206. The temperature measuring optical fiber 8 is connected to an optical fiber temperature measuring module 206 in the laser ablation unit 200; and the optical fiber temperature measuring module 206 is connected to a temperature correction module 2011 in the control host 201. In other words, in the optical fiber temperature measuring module 206, the acquired optical signal is fed back to an optical signal decoder in real time, and after signal conversion, the temperature values are fed back to the temperature correction module 2011 in the control host 201.

In the temperature correction module 2011, at least the following processing is performed: ① directly taking a real-time temperature value of the tissue acquired by the optical fiber temperature measuring module 206 in real time as a reference temperature measurement value of the magnetic resonance guidance unit 100; ② based on the reference temperature measurement value, generating a corrected temperature image by the magnetic resonance guidance unit 100; and ③ feeding back image correction information to the control host 201 from the temperature correction module 201, where the image correction information includes at least replacing the real-time temperature image with the corrected temperature image. The control host 201 is configured to perform real-time regulation on the laser module 202 according to the corrected temperature image.

Further, in some embodiments, the control host 201 is configured to synchronously or asynchronously regulate laser operation parameters (including the laser output power, the light emitting time, the light emitting mode and the like) of the first laser device 2021 and the second laser device 2022 according to the surgical plan and the corrected temperature image.

Further, the temperature measuring optical fiber 8 may be further configured to feed back a safety parameter condition of the system to the control host 201, and when the temperature data measured by the temperature measuring optical fiber 8 exceeds a safety threshold, the control host 201 emergently stops operation of the laser module.

In some embodiments: a first optimization scheme for system temperature control design is provided.

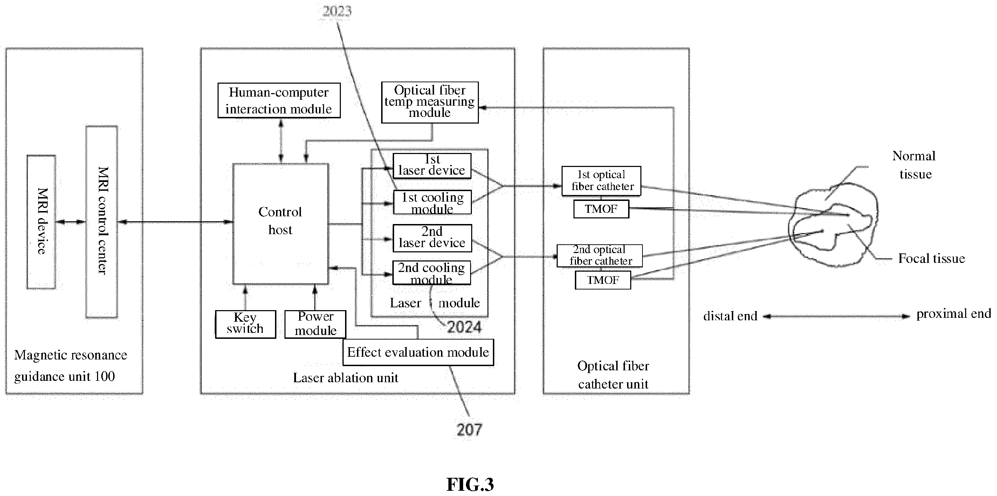

As shown in FIG. 3, in order to further improve the precision of conformal ablation, on the basis of the first embodiment, the laser module 202 is further configured with a cooling module connected to both the control host 201 and the optical fiber catheter unit 300. The control host 201 controls operation of the cooling module; and the cooling module is configured to cool the optical fiber catheter unit 300 and a surrounding tissue of the optical fiber catheter unit 300 by driving and controlling circulatory flow of a cooling medium. The optical fiber catheter unit 300 has the first optical fiber catheter 301 and the second optical fiber catheter 302 of the same structure, and the first optical fiber catheter 301 is taken as an example for illustration: the first optical fiber catheter 301 includes an optical fiber, a cooling inner tube and a cooling outer tube. The optical fiber is connected to the first laser device 2021; and a circulating inlet and outlet channel for the cooling medium formed by the optical fiber, the cooling inner tube and the cooling outer tube is connected to a first cooling module.

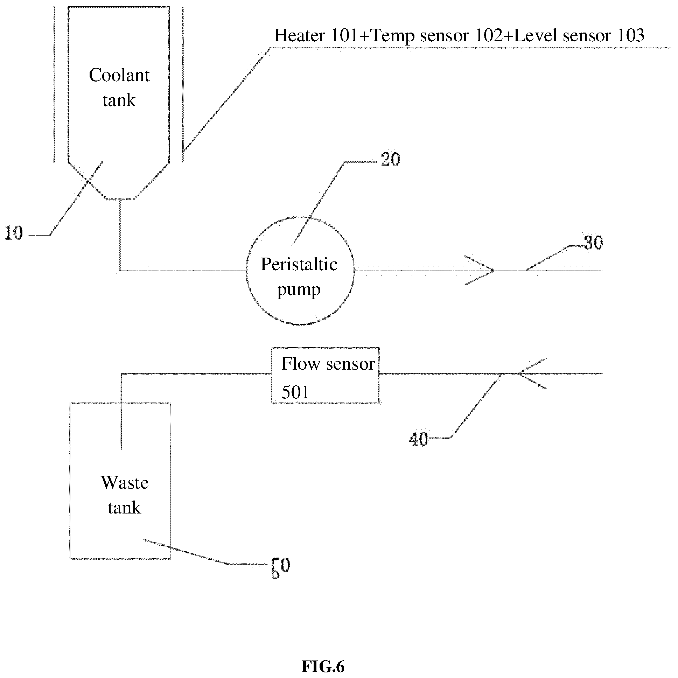

The cooling module includes a first cooling module 2023 and a second cooling module 2024 of the same structure. The first cooling module 2023 is configured to cool the first optical fiber catheter 301, and the second cooling module 2024 is configured to cool the second optical fiber catheter 302. Referring also to FIG. 6, the first cooling module 2023 includes a coolant tank 10, a peristaltic pump 20, a water inlet pipe 30, a water return pipe 40, and a waste tank 50. The coolant tank 10 is further provided with a heater 101, a temperature sensor 102, and a level sensor 103, and the water return pipe 40 is further provided with a flow sensor 501. In some embodiments, the first cooling module 2023 adopts a single-channel circulation system (with no additional circulation pipeline between the waste tank 50 and the coolant tank 10), to prevent circulating reflux contamination.

When the first cooling module 2023 is in operation, the heater 101 heats the coolant in the coolant tank 10 to a suitable temperature, for example 37.2 °C. The temperature in the coolant tank 10 is monitored and detected by the temperature sensor 102, and then the peristaltic pump 20 is started to deliver the coolant into the first optical fiber catheter 301 to form a cooling loop. Then, the coolant enters the waste tank 50 through the water return pipe 40 where a flow sensor 501 is additionally provided. The flow sensor 501 is configured to confirm whether the coolant is normally returned. In addition, the level sensor 103 may detect a level of the coolant in the coolant tank 10, and send an alarm signal when the coolant is insufficient, to request replacement or replenishment of the coolant. In some embodiments, the coolant adopts a water cooling mode, which have a more stable cold source, a better effect and a lower preparation cost. The coolant tank 10 may be directly adapted to a conventional normal saline bottle without any additional switching structure, and when the coolant is to be replaced, the normal saline bottle can be simply placed in the coolant tank 10 and connected to a water delivery pipe.

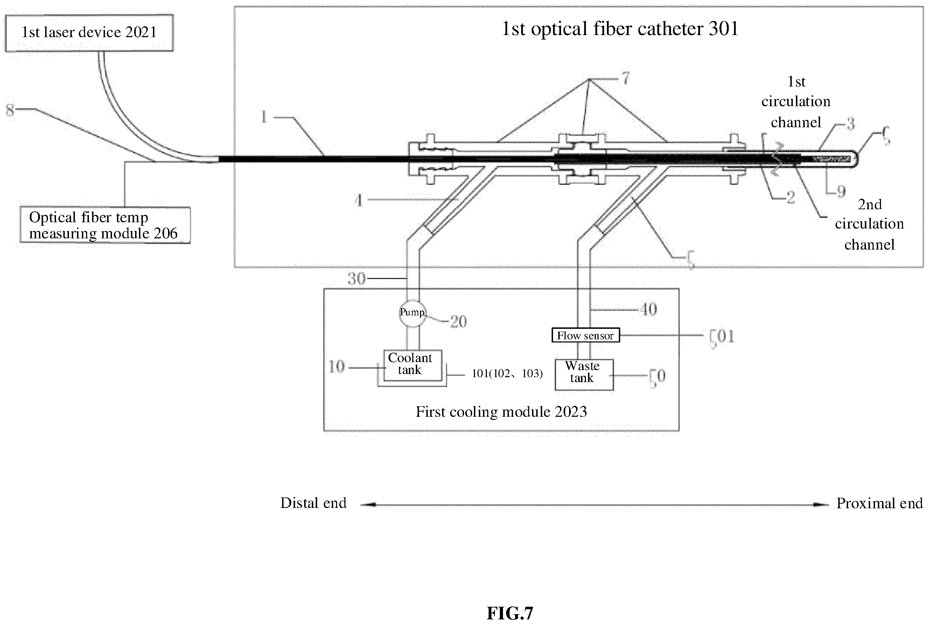

Referring also to FIG. 7, the first optical fiber catheter 301 may generally have a structure as follows: the first optical fiber catheter 301 includes an optical fiber 1, a cooling inner tube 2, and a cooling outer tube 3. The optical fiber 1 is located in the cooling inner tube 2, and an axial clearance between an outer wall of the optical fiber 1 and an inner wall of the cooling inner tube 2 forms a first circulation channel. The cooling inner tube 2 is disposed within the cooling outer tube 3, and an axial clearance between an inner wall of the cooling outer tube 3 and an outer wall of the cooling inner tube 2forms a second circulation channel. A proximal end of the first circulation channel and a proximal end of the second circulation channel are communicated at a proximal end of the cooling outer tube 3 to form a backflow cavity 6. A distal end of the first circulation channel is in communication with the water inlet pipe 30, and a distal end of the second circulation channel is in communication with the water return pipe 40.

Referring again to FIG. 7, the first optical fiber catheter 301 further includes a fastening assembly 7 configured to fasten and connect the optical fiber 1, the inner tube 2, and the outer tube 3, and meanwhile, the fastening assembly 7 has a coolant inlet 4 in communication with the first circulation channel, and a coolant outlet 5 in communication with the second circulation channel. The coolant inlet 4 is in communication with the water inlet pipe 30, and the coolant outlet 5 is in communication with the water return pipe 40.

The optical fiber catheter and the fastening assembly belong to existing art, and thus the specific structural characteristics thereof will not be described in detail here.

In some embodiments, the control host 201 acquires and processes feedback information of various modules in the multi-wavelength laser system during a surgical procedure, and outputs signals to the modules, while controlling all the functional modules to work together in a coordinated manner. In this manner, real-time temperature detection, ablation condition evaluation, and human-computer interaction can be completed, and automatic adjustment of the laser power and the efficiency of the cooling module can be implemented. For example, the control host 201 may regulate operation parameters, such as a laser output power, laser output time, a light emitting mode, and a coolant flow rate in real time according to the ablation condition.

Further, the control host 201 may monitor operation parameters of the laser module 202, the optical fiber temperature measuring module 206, and the cooling module in real time; and when an operation parameter exceeds a safe operation threshold value, the control host 201 is configured to stop the laser module 202 and/or the cooling module.

In some embodiments: a second optimization scheme for system temperature control design is provided.

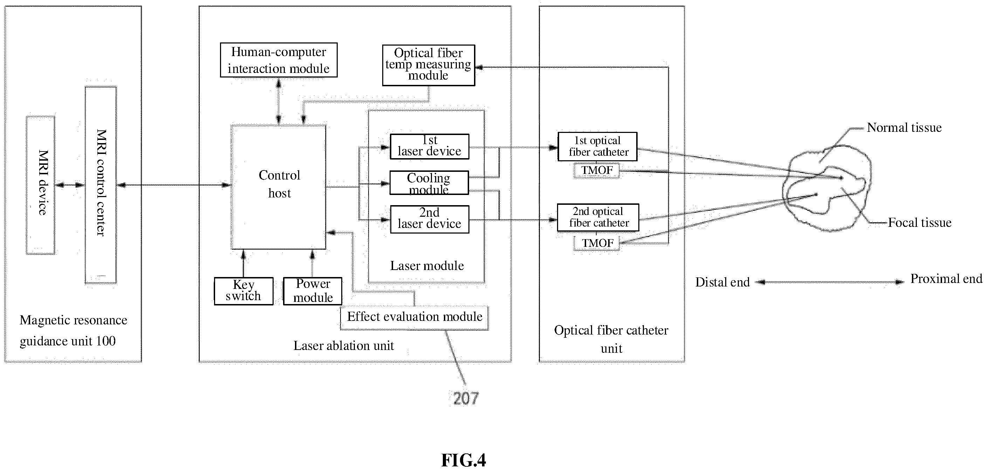

Referring to FIG. 4, the third embodiment differs from the second embodiment merely in that the first optical fiber catheter 301 and the second optical fiber catheter 302 share one cooling module, and the sharing form or manner may be obtained by those skilled in the art according to the existing art, and therefore is not described in detail here.

In some embodiments: a design scheme for overall optimization of the multi-wavelength system is provided.

Referring again to FIG. 3, the fourth embodiment is an optimization based on the first embodiment, the second embodiment or the third embodiment, and therefore only the differences and improvements will be described in detail herein.

The laser ablation unit 200 of the present disclosure mainly includes: a control host 201, a human-computer interaction module 203, a laser module 202, a cooling module, a power module 204, an optical fiber temperature measuring module 206, and an effect evaluation module 207.
(1) The control host 201 is configured to receive a signal and output a control signal, and monitor ablation progress in real time. Therefore, the functions and actions of the control host 201 are mainly embodied in the preoperative and intraoperative stages.

In the preoperative stage: in cooperation with preoperative software, the control host 201 is configured to complete profiling, multi-modal 3D modeling, marking focus and neurovascular locations, planning an appropriate surgical path, and generating a surgical plan for a patient according to medical image information of the patient. The surgical plan contains insertion path planning for an optical fiber catheter, estimated conditions of a region to be ablated, the used laser power, light emitting time, light emitting mode, number of desired ablation channels, and the like.

In the intraoperative stage: the control host 201 can process digital image information transmitted from the MRI control center 102 in time, generate a real-time temperature image of a focus area through an MRI temperature imaging technique and a multi-modal fusion technique, and display the real-time temperature image on a human-computer interaction module 203. In the intraoperative stage, the control host 201 may monitor ablation conditions of the focus area in real time according to the surgical plan and the real-time temperature image, and regulate the laser output power, laser output time, light emitting mode, and the like of the first laser device 2021 and the second laser device 2022 synchronously or asynchronously according to the ablation data displayed on the human-computer interaction module 203.

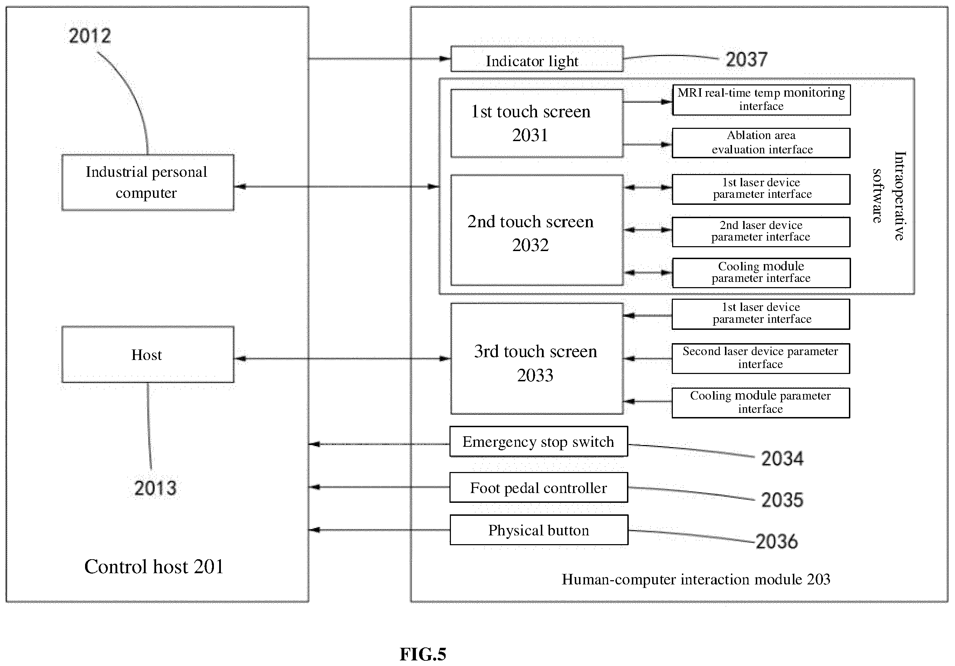

(2) The human-computer interaction module 203 is connected to the control host 201, and configured to receive input of the laser ablation operation parameters and display real-time information. Referring also to FIG. 5, the human-computer interaction module 203 is used with the control host, and displays in cooperation with a plurality of touch screens, physical buttons and working indicator lights. Meanwhile, a plurality of input modes, such as a touch screen, a mouse, a keyboard, a solid knob and the like, are provided. In this manner, when one part fails, the other part can be used instead for controlling, thereby increasing the stability and safety of the multi-wavelength laser system. Specifically, the human-computer interaction module 203 includes a first touch screen 2031, a second touch screen 2032, a third touch screen 2033, an emergency stop switch 2034, a foot pedal controller 2035, a physical button 2036, and an indicator light 2037. The first touch screen 2031 and the second touch screen 2032 are each connected to an industrial personal computer 2012 of the control host 201, while the third touch screen 2033 is connected to a motherboard 2013 of the control host 201.

Further, the first touch screen 2031 includes an MRI real-time temperature monitoring interface and an ablation area evaluation interface; the second touch screen 2032 includes a first laser device parameter interface, a second laser device parameter interface, and a cooling module parameter interface; and the third touch screen and the second touch screen display the same or different interface contents. Because the motherboard 2013 has higher stability and safety than the industrial personal computer 2012, when the second touch screen 2032 fails, the ablation can be continued according to the display contents on the third touch screen 2033.

Further, the emergency stop switch 2034 is configured to stop the laser module, the optical fiber catheter, the cooling module, and the like in emergency, but not the human-computer interaction module 203. The foot pedal controller 2035 is configured to control laser emission of the laser module 202, and the switching between lasers of different wavelengths and the switching of the light emitting mode. The control host 201 is configured to monitor operation parameters of the laser module 202, the optical fiber temperature measuring module 206, and the cooling module in real time; and when an operation parameter exceeds a safe operation threshold value, the control host 201 is configured to emergently stop the laser module 202 and/or the cooling module.

(3) The laser module 202 is configured to emit a plurality of lasers of the same energy or different energies. In this embodiment, the laser module 202 includes a first laser device 2021 and a second laser device 2022. The first laser device 2021 can generate lasers of a first wavelength, and the second laser device 2022 can generate lasers of a second wavelength. The first wavelength and the second wavelength may be the same or different. In some embodiments of the present disclosure, the first laser device 2021 is configured to generate lasers of a first wavelength 980nm, and the second laser device 2022 is configured to generate lasers of a second wavelength 1064nm. In an ablation surgery, the control host 201 is configured to regulate the first laser device 2021 and the second laser device 2022 to emit laser simultaneously, or regulate only one of the first laser device 2021 or the second laser device 2022 to emit lasers; or may send a regulation instruction that indicates the first laser device 2021 and the second laser device 2022 to emit light in a specified order according to the surgical requirement. The regulation instruction may include a laser light emitting power, light emitting time, a light emitting mode, synchronous light emitting, asynchronous light emitting, a light emitting angle or the like.

Further, a semiconductor laser is used at the wavelength 980nm, and a semiconductor laser or Nd:YAG laser is use at the wavelength 1064nm. The laser of the wavelength 980nm and the laser of the wavelength 1064nm are both high-power lasers commonly used for ablation. Compared with the laser of the wavelength 980nm, the laser of the wavelength 1064nm is more penetrating, and therefore can be used for a larger volume of ablation. As can be seen, the multi-wavelength laser system of the present disclosure can generate multiple different bands of lasers, which can be further matched with optical fiber catheters correspondingly connected thereto to implement light emitting ablation, and further implement precise conformal ablation on a regular or irregular tumor. The multi-wavelength laser system of the present disclosure can be used without considering a diameter or shape characteristics of the tumor and completely regardless of the size, shape or position, which, compared with the existing art, can greatly increase the application range of LITT, and bring about more flexible ablation schemes, and is worthy of being popularized in the field.

Apparently, the laser module 202 may be configured with only one laser device, but the laser device is configured to emit lasers of multiple wavelengths, where each wavelength is matched with a corresponding optical fiber catheter, so that multi-wavelength light emission and ablation can be implemented. Alternatively, the laser module 202 may be configured with at least two laser devices which can emit lasers of the same band. The implementation of these improvements can be achieved according to the existing art, and will not be described in detail herein.

(4) The cooling module is configured to cool the optical fiber catheter unit 300 and a surrounding tissue of the optical fiber catheter unit 300 by driving and controlling circulatory flow of a cooling medium. The specific implementation of the cooling module may refer to the contents of the second embodiment of the present disclosure.

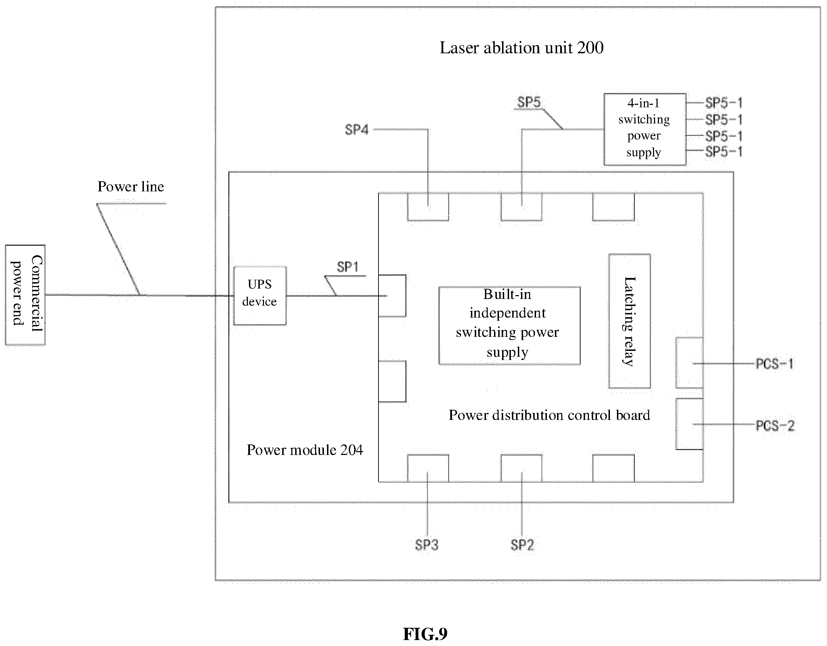

(5) The power module 204 is configured to provide stable power support for each of the other modules. Referring to FIG. 9, a schematic diagram showing power distribution of a power module in a laser ablation unit is shown. The power module 204 is configured to provide power for each module in the laser ablation unit 200, for example, the control host 201, the human-computer interaction module 203, the laser module 202, the optical fiber temperature measuring module 206, the effect evaluation module 207, and the like. The power module 204 mainly includes at least one UPS device and at least one power distribution control board. An electric energy input end of the laser ablation unit 200 is connected to a power line. The power line has one end connected to a commercial power end and the other end connected to the UPS device. In other words, an output end of the power line is connected to an electric energy input end of the UPS device. The UPS device is in a charging state when the commercial power end supplies power normally, and once the commercial power end or the power line breaks down or is interrupted, the UPS device immediately outputs the stored electric energy to the laser ablation unit 200 so that the power supply continuity and normal use of each module in the laser ablation unit 200 are ensured. In some embodiments, the UPS device may maintain power supply for about 15min.

In addition, circuit connection wires of the modules in the laser ablation unit 200 are all centralized on the power distribution control board. This has the advantages of reasonable distribution of circuit management for the modules in the laser ablation unit 200, clear and definite wiring, easy maintenance, and contribution to further reducing the occupied space of the laser ablation unit 200. Furthermore, the power distribution control board is provided with a plurality of connection terminals, a built-in independent switching power supply, and a latching relay. The connection terminals are respectively and electrically connected to at least five electric energy output channels, including an SP1 channel, an SP2 channel, an SP3 channel, an SP4 channel, and an SP5 channel. The SP1 channel, the SP2 channel, the SP3 channel, the SP4 channel, and the SP5 channel may be electrically connected to the modules in the laser ablation unit 200, respectively. For example, the SP1 channel is electrically connected to the UPS device and serves as a total input of the entire board power supply, the SP2 channel is connected to the first touch screen 2031, the SP3 channel is connected to the second touch screen 2032, the SP4 channel is connected to the control host 201, and the SP5 channel is connected to a 4-in-1 switching power supply.

In some embodiments, the 4-in-1 switching power supply is provided with at least four electric energy output channels, including a Power-1 channel, a Power-2 channel, a Power-3 channel, and a Power-4 channel. The four electric energy output channels may output the same direct-current power supply or different direct current power supplies, and the Power-1 channel, the Power-2 channel, the Power-3 channel, and the Power-4 channel are operated independent of one another without any interference, thereby guaranteeing the power utilization safety.

In some embodiments of the present disclosure, the built-in independent switching power supply is electrically connected to the latching relay. In some embodiments, the latching relay is electrically connected to the SP5 channel. Meanwhile, the latching relay is further connected to a PCS-1 channel configured to electrically connect to the emergency stop control switch 2034 and the key switch 205. The power distribution control board further includes a PCS-2 channel connected to the cooling module. The advantages of such wiring lie in that strong electricity can be controlled by weak electricity, the power utilization safety of the device is increased while functional reliability of the device is guaranteed, and the latching relay can maintain a last operating state of the device so that when the start button needs to be re-pressed when the device is resumed to normal operation from an emergency stop, the electrical safety problem caused by false resuming after an emergency stop can be avoided.

(6) The optical fiber temperature measuring module 206 is configured to acquire and process real-time temperatures of the optical fiber catheter unit 300 and a surrounding tissue of the optical fiber catheter unit 300 transmitted from the temperature measuring optical fiber 8; and assist the control host 201 to generate an accurate corrected temperature image, to achieve real-time intraoperative temperature feedback and more accurate ablation actions. For more details, please refer to the first embodiment of the present disclosure.

(7) The effect evaluation module 207 is configured to make real-time intraoperative estimates of a tissue ablation condition using an Arrhenius model or a CEM43 model; and the control host 201 is configured to generate in real time, according to ablation progress fed back by the effect evaluation module 207, a regulation instruction including at least one of: a laser output power, light emitting time, a light emitting mode, or a coolant flow rate.

Furthermore, the multi-wavelength laser system further includes other peripheral interfaces, such as a USB interface, a safety switch interface, a network cable, an optical drive and the like, to ensure normal operation of the system.

Further, the multi-wavelength laser system further includes software, where the software performs functions including at least one of:
① surgical plan generation, where the surgical plan contains information corresponding to each of the N laser devices, where the information includes at least one of: a planned ablation area and/or volume, a laser power, light emitting time or a light emitting mode used for achieving a preset ablation result, the number of desired ablation channels, or a coolant flow rate;
② real-time control, including under the guidance of the magnetic resonance guidance unit 100 and according to the surgical plan and the corrected temperature image corresponding to each of the N laser devices, regulating operation parameters of each laser device in an operating state and the cooling module in real time, and performing ablation monitoring in real time; or
③ comparison and analysis, including comparing and analyzing, by Boolean operation for example, information in the surgical plan corresponding to each laser device with post-operative information of the laser device, and generating ablation result information according to a comparison result and displaying the ablation result information on the human-computer interaction module 203; where the compared contents include: a planned ablation area and/or volume, and an actual post-operative ablation area and/or volume; and the ablation result information includes at least one of: an ablation area percentage, an ablation volume percentage, an ablation area percentage, or a comparison diagram of before and after ablation.

The above are merely embodiments of the present disclosure, and are not intended to limit the scope of the present disclosure. The invention is defined in the appended claims.

## Claims

1. A multi-wavelength laser system for thermal ablation in neurosurgery, comprising:
a magnetic resonance guidance unit (100) including a magnetic resonance imaging, MRI, device (101) and an MRI control center (102); wherein the MRI control center (102) is configured to perform processing including at least one of: data acquisition, data processing, image reconstruction, image display, or image storage;
a laser ablation unit (200) connected to the magnetic resonance guidance unit (100) and including a control host (201); wherein the control host (201) is configured to complete, according to digital image information of a patient, at least one of: profiling, 3D modeling, or generating a surgical plan of the patient; and the control host (201) is configured to fuse the digital image information through an MRI temperature imaging technique to generate a real-time temperature image, and display the real-time temperature image on a human-computer interaction module (203); wherein
the laser ablation unit (200) further includes a laser module (202) connected to the control host (201); and
an optical fiber catheter unit (300) connected to the laser ablation unit (200);
**characterized in that** the laser module (202) comprises a first laser device (2021) and a second laser device (2022); and the control host (201) is configured to synchronously or asynchronously regulate laser operation parameters of the first laser device (2021) and the second laser device (2022) according to the surgical plan and the real-time temperature image;
the optical fiber catheter unit (300) comprises a first optical fiber catheter (301) and a second optical fiber catheter (302); and the first optical fiber catheter (301) is connected to the first laser device (2021), and the second optical fiber catheter is connected to the second laser device (2022);
the first laser device (2021) is configured to emit a laser beam of a first wavelength, and the second laser device (2022) is configured to emit a laser beam of a second wavelength;
the first wavelength is 980nm, and the second wavelength is 1064nm.

2. The multi-wavelength laser system for thermal ablation in neurosurgery of claim 1, wherein
at least one of the first optical fiber catheter (301) or the second optical fiber catheter (302) is provided with a temperature measuring optical fiber (8) configured to detect real-time temperatures of the optical fiber catheter unit (300), where the temperature measuring optical fiber (8) is located,
and a surrounding tissue of the optical fiber catheter unit (300); the temperature measuring optical fiber (8) is connected to an optical fiber temperature measuring module (206) in the laser ablation unit (200); and the optical fiber temperature measuring module (206) is connected to a temperature correction module (2011) in the control host (201) for temperature correction.

3. The multi-wavelength laser system for thermal ablation in neurosurgery of claim 2, wherein
the temperature correction module (2011) is configured to complete processing including at least: taking a real-time temperature value of the tissue acquired by the optical fiber temperature measuring module (206) in real time as a reference temperature measurement value of the magnetic resonance guidance unit (100), and feeding back a corrected temperature image to the control host (201);
wherein the corrected temperature image is generated by the magnetic resonance guidance unit (100) based on the reference temperature measurement value; and
the corrected temperature image fed back to the control host (201) is used to replace the real-time temperature image.

4. The multi-wavelength laser system for thermal ablation in neurosurgery of claim 3, wherein
the laser module (202) is further configured with a cooling module connected to the control host (201) and the optical fiber catheter unit (300); and
the control host (201) is configured to control operation of the cooling module such that the cooling module cools the optical fiber catheter unit (300) and a surrounding tissue of the optical fiber catheter unit (300) by driving and controlling flow of a cooling medium.

5. The multi-wavelength laser system for thermal ablation in neurosurgery of claim 4, wherein
the laser ablation unit (200) includes a power module (204) and an effect evaluation module (207).

6. The multi-wavelength laser system for thermal ablation in neurosurgery of claim 5, wherein
the power module (204) has at least one UPS device and at least one power distribution control board; wherein an electric energy input end of the laser ablation unit (200) is connected to a power line; and the power line has one end connected to a commercial power end and the other end connected to the electric energy input end of the UPS device.

7. The multi-wavelength laser system for thermal ablation in neurosurgery of claim 6, wherein
the power distribution control board includes a plurality of connection terminals, a built-in independent switching power supply, and a latching relay; the latching relay is electrically connected to a PCS-1 channel that is configured to be electrically connected to an emergency stop switch (2034) and a key switch (205); and the power distribution control board further includes a PCS-2 channel connected to the cooling module.

8. The multi-wavelength laser system for thermal ablation in neurosurgery of claim 5, wherein
the effect evaluation module (207) is configured to make real-time intraoperative estimates of a tissue ablation condition using an Arrhenius model or a CEM43 model; and
the control host (201) is configured to generate in real time, according to ablation progress fed back by the effect evaluation module (207), a regulation instruction including at least one of: a laser output power, light emitting time, a light emitting mode, or a coolant flow rate.

9. The multi-wavelength laser system for thermal ablation in neurosurgery of claim 5, wherein
the control host (201) is configured to monitor safe operation parameters of the laser module (202), the optical fiber temperature measuring module (206) and the cooling module in real time; and when an operation parameter exceeds a safe operation threshold value, the control host (201) is configured to control either or both of the laser module (202) and the cooling module to stop emitting light.

10. The multi-wavelength laser system for thermal ablation in neurosurgery of any one of claims 4 to 9, further comprising: software, wherein when the software is executed by a processor, the system performs functions including at least one of:
surgical plan generation, wherein the surgical plan contains information corresponding to each of the laser devices, wherein the information includes at least one of: a planned ablation area, a planned ablation volume, a laser power, light emitting time or a light emitting mode used for achieving a preset ablation result, a coolant flow rate, or insertion path planning for an optical fiber catheter;
real-time control, including under the guidance of the magnetic resonance guidance unit (100) and according to the surgical plan and the corrected temperature image corresponding to each of the laser devices, regulating operation parameters of each laser device in an operating state and the cooling module in real time, and performing ablation monitoring in real time; or
comparison and analysis, including comparing information in the surgical plan corresponding to each laser device with post-operative information of the laser device, and generating ablation result information according to a comparison result and displaying the ablation result information on the human-computer interaction module (203); wherein the compared contents include: a planned ablation area or volume, and an actual post-operative ablation area or volume; and the ablation result information includes at least one of: an ablation area percentage, an ablation volume percentage, or a comparison diagram of before and after ablation.

## Patentansprüche

1. Ein Mehrwellenlängen-Lasersystem zur thermischen Ablation in Neurochirurgie, umfassend:
eine Magnetresonanz-Führungseinheit (100) mit einem Magnetresonanztomographie(MRT)-Gerät (101) und einer MRT-Steuerzentrale (102); wobei die MRT-Steuerzentrale (102) dazu konfiguriert ist, Verarbeitung durchzuführen, die mindestens einen der Folgenden umfasst: Datenerfassung, Datenverarbeitung, Bildrekonstruktion, Bildanzeige oder Bildspeicherung;
eine Laserablationseinheit (200), die mit der Magnetresonanz-Führungseinheit (100) verbunden ist und einen Steuerungshost (201) umfasst; wobei der Steuerungshost (201) dazu konfiguriert ist, gemäß digitaler Bildinformation eines Patienten mindestens eine der Folgenden auszuführen: Profilerstellung, 3D-Modellierung oder Erzeugung eines Operationsplans für den Patienten; und der Steuerungshost (201) dazu konfiguriert ist, die digitale Bildinformation durch MRT-Temperaturbildgebungstechnik zusammenzuführen, um ein Echtzeit-Temperaturbild zu erzeugen, und das Echtzeit-Temperaturbild auf einem Mensch-Computer-Interaktionsmodul (203) anzuzeigen; wobei
die Laserablationseinheit (200) ferner ein Lasermodul (202) umfasst, das mit dem Steuerungshost (201) verbunden ist; und
eine mit der Laserablationseinheit (200) verbundene Glasfaserkathetereinheit (300);
**dadurch gekennzeichnet, dass** das Lasermodul (202) eine erste Laservorrichtung (2021) und eine zweite Laservorrichtung (2022) umfasst; und der Steuerungshost (201) dazu konfiguriert ist, die Laserbetriebsparameter der ersten Laservorrichtung (2021) und der zweiten Laservorrichtung (2022) gemäß dem Operationsplan und dem Echtzeit-Temperaturbild synchron oder asynchron zu regeln;
die Glasfaserkathetereinheit (300) einen ersten Glasfaserkatheter (301) und einen zweiten Glasfaserkatheter (302) umfasst; und der erste Glasfaserkatheter (301) mit der ersten Laservorrichtung (2021) verbunden ist, und der zweite Glasfaserkatheter mit der zweiten Laservorrichtung (2022) verbunden ist;
die erste Laservorrichtung (2021) dazu konfiguriert ist, einen Laserstrahl einer ersten Wellenlänge auszustrahlen, und die zweite Laservorrichtung (2022) dazu konfiguriert ist, einen Laserstrahl einer zweiten Wellenlänge auszustrahlen;
die erste Wellenlänge 980 nm beträgt, und die zweite Wellenlänge 1064 nm beträgt.

2. Das Mehrwellenlängen-Lasersystem zur thermischen Ablation in Neurochirurgie nach Anspruch 1, wobei mindestens einer von dem ersten Glasfaserkatheter (301) oder dem zweiten Glasfaserkatheter (302) mit einer Temperaturmessungsglasfaser (8) versehen ist, die dazu konfiguriert ist, Echtzeit-Temperaturen der Glasfaserkathetereinheit (300), wo sich die Temperaturmessungsglasfaser (8) befindet, sowie des umgebenden Gewebes der Glasfaserkathetereinheit (300) zu erfassen; die Temperaturmessungsglasfaser (8) mit einem Glasfaser-Temperaturmessungsmodul (206) in der Laserablationseinheit (200) verbunden ist; und das Glasfaser-Temperaturmessungsmodul (206) zur Temperaturkorrektur mit einem Temperaturkorrekturmodul (2011) im Steuerungshost (201) verbunden ist.

3. Das Mehrwellenlängen-Lasersystem zur thermischen Ablation in Neurochirurgie nach Anspruch 2, wobei das Temperaturkorrekturmodul (2011) dazu konfiguriert ist, eine Verarbeitung auszuführen, die mindestens umfasst: Erfassung eines durch das Glasfaser-Temperaturmessungsmodul (206) in Echtzeit ermittelten Echtzeit-Temperaturwerts des Gewebes als Referenz-Temperaturmessungswert der Magnetresonanz-Führungseinheit (100), und Rückführung eines korrigierten Temperaturbildes an den Steuerungshost (201);
wobei das korrigierte Temperaturbild durch die Magnetresonanz-Führungseinheit (100) auf der Grundlage des Referenz-Temperaturmessungswerts erzeugt ist; und
das an den Steuerungshost (201) zurückgeführte korrigierte Temperaturbild dazu verwendet ist, um das Echtzeit-Temperaturbild zu ersetzen.

4. Das Mehrwellenlängen-Lasersystem zur thermischen Ablation in Neurochirurgie nach Anspruch 3, wobei das Lasermodul (202) ferner mit einem Kühlungsmodul ausgestattet ist, das mit dem Steuerungshost (201) und der Glasfaserkathetereinheit (300) verbunden ist; und
der Steuerungshost (201) dazu konfiguriert ist, den Betrieb des Kühlungsmoduls zu steuern, so dass das Kühlungsmodul die Glasfaserkathetereinheit (300) und das umgebende Gewebe der Glasfaserkathetereinheit (300) durch Ansteuerung und Steuerung von Fluss eines Kühlmediums kühlt.

5. Das Mehrwellenlängen-Lasersystem zur thermischen Ablation in Neurochirurgie nach Anspruch 4, wobei die Laserablationseinheit (200) ein Stromversorgungsmodul (204) und ein Wirkungsbewertungsmodul (207) umfasst.

6. Das Mehrwellenlängen-Lasersystem zur thermischen Ablation in Neurochirurgie nach Anspruch 5, wobei das Stromversorgungsmodul (204) mindestens eine USV-Einrichtung und mindestens eine Stromversorgungsverteilungssteuerungsplatine aufweist; wobei ein elektrischer Energieeingang der Laserablationseinheit (200) mit einer Stromleitung verbunden ist; und die Stromleitung mit einem Ende an einen Netzanschluss und mit dem anderen Ende an den elektrischen Energieeingang der USV-Einrichtung angeschlossen ist.

7. Das Mehrwellenlängen-Lasersystem zur thermischen Ablation in Neurochirurgie nach Anspruch 6, wobei die Stromversorgungsverteilungssteuerungsplatine eine Vielzahl von Anschlussklemmen, eine integrierte unabhängige Schaltstromversorgung und ein Verriegelungsrelais umfasst; wobei das Verriegelungsrelais elektrisch mit einem PCS-1-Kanal verbunden ist, der dazu konfiguriert ist, elektrisch mit einem Not-Aus-Schalter (2034) und einem Schlüsselschalter (205) verbunden ist; und wobei die Stromversorgungsverteilungssteuerungsplatine ferner einen mit dem Kühlungsmodul verbundenen PCS-2-Kanal umfasst.

8. Das Mehrwellenlängen-Lasersystem zur thermischen Ablation in Neurochirurgie nach Anspruch 5, wobei das Wirkungsbewertungsmodul (207) dazu konfiguriert ist, unter Verwendung eines Arrhenius-Modells oder eines CEM43-Modells intraoperative Echtzeit-Schätzungen eines Gewebeablationszustands vorzunehmmen; und
der Steuerungshost (201) dazu konfiguriert ist, in Echtzeit gemäß dem durch das Wirkungsbewertungsmodul (207) rückgemeldeten Ablationsfortschritt einen Regelbefehl zu erzeugen, der mindestens eines der Folgenden umfasst: eine Laserausgangsleistung, eine Lichtemissionszeit, einen Lichtemissionsmodus oder eine Kühlmediumdurchflussrate.

9. Das Mehrwellenlängen-Lasersystem zur thermischen Ablation in Neurochirurgie nach Anspruch 5, wobei der Steuerungshost (201) dazu konfiguriert ist, sichere Betriebsparameter des Lasermoduls (202), des Glasfasertemperaturmessungsmoduls (206) und des Kühlungsmoduls in Echtzeit zu überwachen; und wenn ein Betriebsparameter einen Schwellenwert für den sicheren Betrieb überschreitet, der Steuerungshost (201) dazu konfiguriert ist, entweder das Lasermodul (202) oder das Kühlungsmodul oder beide zu steuern, so dass die Lichtemission gestoppt wird.

10. Das Mehrwellenlängen-Lasersystem zur thermischen Ablation in Neurochirurgie nach einem der Ansprüche 4 bis 9, das ferner umfasst: Software, wobei das System, wenn die Software durch einen Prozessor durchgeführt wird, Funktionen ausführt, die mindestens eine der Folgenden umfassen:
Erstellung eines Operationsplans, wobei der Operationsplan Informationen enthält, die jeder der Laservorrichtungen entsprechen, wobei die Informationen mindestens eines der Folgenden umfassen: einen geplanten Ablationsbereich, ein geplantes Ablationsvolumen, eine Laserleistung, eine Lichtemissionszeit oder einen Lichtemissionsmodus, der zum Erreichen eines voreingestellten Ablationsergebnisses verwendet ist, eine Kühlmediumdurchflussrate oder eine Einsteckwegplanung für einen Glasfaserkatheter;
Echtzeit-Steuerung, umfassend Regelung von Betriebsparametern jeder Laservorrichtung im Betriebszustand und des Kühlungsmoduls in Echtzeit unter der Führung der Magnetresonanz-Führungseinheit (100) und gemäß dem Operationsplan und dem korrigierten Temperaturbild, das jeder der Laservorrichtungen entspricht, und Durchführung einer Ablationsüberwachung in Echtzeit; oder
Vergleichung und Analysierung, umfassend Vergleichung von Informationen im Operationsplan, die jeder Laservorrichtung entsprechen, mit postoperativen Informationen der Laservorrichtung, und Erzeugung von Informationen über Ablationsergebnis gemäß einem Vergleichungssergebnis und Anzeigung der Informationen über Ablationsergebnis auf dem Mensch-Computer-Interaktionsmodul (203); wobei die verglichenen Inhalte umfassen: einen geplanten Ablationsbereich oder ein geplantes Ablationsvolumen, und einen tatsächlichen postoperativen Ablationsbereich oder ein tatsächliches postoperatives Ablationsvolumen; und die Informationen über Ablationsergebnis mindestens eines der Folgenden umfassen: einen prozentualen Anteil des Ablationsbereichs, einen prozentualen Anteil des Ablationsvolumens oder ein Vergleichungssdiagramm von vor und nach der Ablation.

## Revendications

1. Système laser multi-longueurs d'onde pour l'ablation thermique en neurochirurgie, comprenant:
une unité de guidage par résonance magnétique (100) comportant un dispositif d'imagerie par résonance magnétique (IRM) (101) et un centre de commande IRM (102); dans lequel le centre de commande IRM (102) est configuré pour effectuer un traitement comprenant au moins l'une des opérations suivantes: acquisition de données, traitement de données, reconstruction d'image, affichage d'image ou stockage d'image;
une unité d'ablation laser (200) connectée à l'unité de guidage par résonance magnétique (100) et comportant un ordinateur hôte de commande (201); dans lequel l'ordinateur hôte de commande (201) est configuré pour réaliser, à partir d'informations d'image numérique d'un patient, au moins l'une des opérations suivantes: profilage, modélisation 3D ou génération d'un plan chirurgical du patient; et l'ordinateur hôte de commande (201) est configuré pour fusionner les informations d'image numérique par une technique d'imagerie de température IRM afin de générer une image de température en temps réel, et pour afficher l'image de température en temps réel sur un module d'interaction homme-machine (203); dans lequel
l'unité d'ablation laser (200) comprend en outre un module laser (202) connecté à l'ordinateur hôte de commande (201); et
une unité de cathéter à fibre optique (300) connectée à l'unité d'ablation laser (200);
**caractérisé en ce que** le module laser (202) comprend un premier dispositif laser (2021) et un deuxième dispositif laser (2022); et l'ordinateur hôte de commande (201) est configuré pour réguler de manière synchrone ou asynchrone les paramètres de fonctionnement laser du premier dispositif laser (2021) et du deuxième dispositif laser (2022) sur la base du plan chirurgical et de l'image de température en temps réel;
l'unité de cathéter à fibre optique (300) comprend un premier cathéter à fibre optique (301) et un deuxième cathéter à fibre optique (302); et le premier cathéter à fibre optique (301) est connecté au premier dispositif laser (2021), et le deuxième cathéter à fibre optique est connecté au deuxième dispositif laser (2022);
le premier dispositif laser (2021) est configuré pour émettre un faisceau laser d'une première longueur d'onde, et le deuxième dispositif laser (2022) est configuré pour émettre un faisceau laser d'une deuxième longueur d'onde;
la première longueur d'onde est 980 nm, et la deuxième longueur d'onde est 1064 nm.

2. Système laser multi-longueurs d'onde pour l'ablation thermique en neurochirurgie selon la revendication 1, dans lequel au moins l'un du premier cathéter à fibre optique (301) ou du deuxième cathéter à fibre optique (302) est muni d'une fibre optique de mesure de température (8) configurée pour détecter les températures en temps réel de l'unité de cathéter à fibre optique (300) où se trouve la fibre optique de mesure de température (8) et d'un tissu autour de l'unité de cathéter à fibre optique (300); la fibre optique de mesure de température (8) est connectée à un module de mesure de température par fibre optique (206) dans l'unité d'ablation laser (200); et le module de mesure de température par fibre optique (206) est connecté à un module de correction de température (2011) dans l'ordinateur hôte de commande (201) pour la correction de température.

3. Système laser multi-longueurs d'onde pour l'ablation thermique en neurochirurgie selon la revendication 2, dans lequel le module de correction de température (2011) est configuré pour réaliser un traitement comprenant au moins: prenant une valeur de température en temps réel du tissu acquise en temps réel par le module de mesure de température par fibre optique (206) comme une valeur de mesure de température de référence de l'unité de guidage par résonance magnétique (100), et renvoyant une image de température corrigée à l'ordinateur hôte de commande (201);
dans lequel l'image de température corrigée est générée par l'unité de guidage par résonance magnétique (100) sur la base de la valeur de mesure de température de référence; et
l'image de température corrigée renvoyée à l'ordinateur hôte de commande (201) est utilisée pour remplacer l'image de température en temps réel.

4. Système laser multi-longueurs d'onde pour l'ablation thermique en neurochirurgie selon la revendication 3, dans lequel le module laser (202) est en outre muni d'un module de refroidissement connecté à l'ordinateur hôte de commande (201) et à l'unité de cathéter à fibre optique (300); et
l'ordinateur hôte de commande (201) est configuré pour commander le fonctionnement du module de refroidissement de sorte que le module de refroidissement refroidisse l'unité de cathéter à fibre optique (300) et un tissu autour de l'unité de cathéter à fibre optique (300) en entraînant et contrôlant l'écoulement d'un milieu de refroidissement.

5. Système laser multi-longueurs d'onde pour l'ablation thermique en neurochirurgie selon la revendication 4, dans lequel l'unité d'ablation laser (200) comprend un module d'alimentation (204) et un module d'évaluation d'effet (207).

6. Système laser multi-longueurs d'onde pour l'ablation thermique en neurochirurgie selon la revendication 5, dans lequel le module d'alimentation (204) comprend au moins un dispositif UPS et au moins une carte de contrôle de distribution d'énergie électrique; dans lequel une entrée d'énergie électrique de l'unité d'ablation laser (200) est connectée à une ligne d'alimentation; et la ligne d'alimentation a une extrémité connectée à une extrémité de secteur et l'autre extrémité connectée à l'entrée d'énergie électrique du dispositif UPS.

7. Système laser multi-longueurs d'onde pour l'ablation thermique en neurochirurgie selon la revendication 6, dans lequel la carte de contrôle de distribution d'énergie électrique comprend une pluralité de bornes de connexion, une alimentation à découpage indépendante intégrée, et un relais à verrouillage; le relais à verrouillage est connecté électriquement à un canal PCS-1 qui est configuré pour être connecté électriquement à un interrupteur d'arrêt d'urgence (2034) et à un interrupteur à clé (205); et la carte de contrôle de distribution d'énergie électrique comprend en outre un canal PCS-2 connecté au module de refroidissement.

8. Système laser multi-longueurs d'onde pour l'ablation thermique en neurochirurgie selon la revendication 5, dans lequel le module d'évaluation d'effet (207) est configuré pour réaliser des estimations peropératoires en temps réel d'une condition d'ablation tissulaire à l'aide d'un modèle d'Arrhenius ou d'un modèle CEM43; et
l'ordinateur hôte de commande (201) est configuré pour générer en temps réel, selon la progression de l'ablation renvoyée par le module d'évaluation d'effet (207), une instruction de régulation comprenant au moins l'undes suivants: une puissance de sortie laser, un temps d'émission lumineuse, un mode d'émission lumineuse, ou un débit de liquide de refroidissement.

9. Système laser multi-longueurs d'onde pour l'ablation thermique en neurochirurgie selon la revendication 5, dans lequel l'ordinateur hôte de commande (201) est configuré pour surveiller en temps réel des paramètres de fonctionnement sécuritaires du module laser (202), du module de mesure de température par fibre optique (206) et du module de refroidissement; et lorsqu'un paramètre de fonctionnement dépasse une valeur seuil de fonctionnement sécuritaire, l'ordinateur hôte de commande (201) est configuré pour commander l'un et/ou l'autre du module laser (202) et du module de refroidissement pour qu'ils cessent d'émettre de la lumière.

10. Système laser multi-longueurs d'onde pour l'ablation thermique en neurochirurgie selon l'une quelconque des revendications 4 à 9, comprenant en outre: un logiciel, dans lequel, lorsque le logiciel est exécuté par un processeur, le système réalise des fonctions comprenant au moins l'un des suivants:
génération de plan chirurgical, dans lequel le plan chirurgical contient des informations correspondant à chacun des dispositifs laser, lesdites informations comprenant au moins l'un des suivants: une zone d'ablation planifiée, un volume d'ablation planifié, une puissance laser, un temps d'émission lumineuse ou un mode d'émission lumineuse utilisé pour atteindre un résultat d'ablation prédéfini, un débit de liquide de refroidissement, ou une planification de trajectoire d'insertion pour un cathéter à fibre optique;
contrôle en temps réel, comprenant, sous le guidage de l'unité de guidage par résonance magnétique (100) et sur la base du plan chirurgical et de l'image de température corrigée correspondant à chacun des dispositifs laser, la régulation en temps réel des paramètres de fonctionnement de chaque dispositif laser dans un état de fonctionnement et du module de refroidissement, et la réalisation d'une surveillance d'ablation en temps réel; ou
comparaison et analyse, comprenant la comparaison des informations dans le plan chirurgical correspondant à chaque dispositif laser avec des informations postopératoires dudit dispositif laser et la génération d'informations de résultat d'ablation en fonction d'un résultat de comparaison et l'affichage desdites informations de résultat d'ablation sur le module d'interaction homme-machine (203); dans lequel les éléments comparés comprennent: une zone ou un volume d'ablation planifié, et une zone ou un volume d'ablation postopératoire réel; et les informations de résultat d'ablation comprennent au moins l'un des suivants: un pourcentage de zone d'ablation, un pourcentage de volume d'ablation, ou un diagramme de comparaison avant et après ablation.
